# EUROPÄISCHE PATENTANMELDUNG

(11) **EP 4 046 715 A1**
(43) Veröffentlichungstag der Anmeldung: **24.08.2022**
(21) Anmeldenummer: 21157898.4
(22) Anmeldetag: 18.02.2021
(51) Int. Cl.: B01L 3/00, B01L 7/00

(54) **MIKROFLUIDISCHES SYSTEM AUS EINER GEFALTETEN FOLIE UND HERSTELLUNGSVERFAHREN**

(71) Anmelder: Joanneum Research Forschungsgesellschaft mbH, 8010 Graz (AT)
(72) Erfinder: Stadlober, Barbara, 8044 Graz (AT); Hesse, Jan, 4020 Linz (AT); Haase, Anja, 8010 Graz (AT); Wolf, Christian, 8041 Graz (AT)
(74) Vertreter: Strehl Schübel-Hopf & Partner

(57) **Zusammenfassung**

Ein Folienverbund, der aus einer einzelnen Folie unter Bildung von mehreren geschichteten Folienlagen (1) gefaltet ist und Mikrofluidikstrukturen (5) in unterschiedlichen Ebenen umfasst, wobei die Mikrofluidikstrukturen dadurch ausgebildet sind, dass Folienlagen mit geprägten Vertiefungen durch die jeweils in Schichtungsrichtung benachbarte Folienlage bedeckt sind, und wobei Mikrofluidikstrukturen einer Ebene mit Mikrofluidikstrukturen einer anderen Ebene über Durchgangsöffnungen in mindestens einer Folienlage verbunden sind; ein Verfahren zum Herstellen des Folienverbundes; und die Verwendung des Folienverbundes.

## Beschreibung

### Gebiet der Erfindung

Die vorliegende Erfindung betrifft einen Folienverbund aus einer gefalteten Folie, die ein mikrofluidisches System ausbildet, ein Verfahren zur Herstellung des Folienverbundes sowie Anwendungen des Folienverbundes.

### Stand der Technik

Bauteile mit Mikrofluidikstrukturen sind typische Elemente in vielen Sensorchips und enthalten Mikrokanäle und Mikrokammern, in denen Flüssigkeiten aufgenommen, weitergeleitet, vermischt oder anderweitig verarbeitet werden können. Beispielhafte Anwendungen sind Biosensoren zur schnellen Diagnose von Keimen in Krankenhäusern oder zur schnellen Analytik von Chemikalien in Prozesstechnik, Umweltanalytik und dergleichen. Für die meisten Anwendungen sind die Mikrofluidikstrukturen mit einer Abdeckung verschlossen, die Ein- und Auslassöffnungen enthält.

Ein Lab-on-Chip-System ist ein kleines Minilabor, das beispielsweise für Schnelltests und Analysen direkt beim Patienten oder beim Hausarzt zum Einsatz kommt. Wird ein solches System auf einem Foliensubstrat integriert, spricht man von einem Lab-on-Foil-System. Es soll klein und nach Gebrauch leicht zu entsorgen und deshalb auch billig herzustellen sein. Lab-on-Foil-Systeme erfüllen diese Bedingungen in besonders hohem Maße. Meist werden solche Systeme in der Größe von Objektträgern gefertigt, um in herkömmliche Messaufbauten zu passen. Aufgrund der dadurch beschränkten Größe dieser Systeme sind darin oft nur wenige Funktionen enthalten.

US 2018/0264474 offenbart ein Mikrofluidiksystem für die Gewebezüchtung, das eine gefaltete Struktur mit mehreren Folienlagen umfasst, worin Mikrofluidikstrukturen in den Folienlagen und Durchgangsöffnungen durch die Folienlagen mittels Laser erzeugt werden.

### Durch die Erfindung zu lösende Aufgaben

Der Stand der Technik offenbart jedoch durch Folienfaltung gebildete Mikrofluidiksysteme, in denen die Mikrofluidikstrukturen durch Prägung gebildet werden. Ein Prägeverfahren hat im Vergleich zu Laserverfahren die Vorteile, dass es schnell und effizient durchgeführt werden kann, insbesondere bei der Durchführung als Rolle-zu-Rolle-Verfahren, und dass auf einfache Weise Mikrofluidikstrukturen hergestellt werden können.

Daher ist es die Aufgabe der vorliegenden Erfindung, einen verbesserten Folienverbund mit Mikrofluidikstrukturen mit einem einfacheren Verfahren bereitzustellen.

### Zusammenfassende Darstellung der Erfindung

Die Aufgabe wurde durch Bereitstellen eines Folienverbundes gelöst, der durch Falten einer einzelnen Folie entsteht und der durch Prägen hergestellte Mikrofluidikstrukturen aufweist.

Der Gegenstand der vorliegenden Erfindung ist insbesondere folgendes:
[1] Folienverbund, der aus einer einzelnen Folie unter Bildung von mehreren geschichteten Folienlagen (1) gefaltet ist und Mikrofluidikstrukturen (5) in unterschiedlichen Ebenen umfasst, wobei die Mikrofluidikstrukturen dadurch ausgebildet sind, dass Folienlagen mit geprägten Vertiefungen durch die jeweils in Schichtungsrichtung benachbarte Folienlage bedeckt sind, und wobei Mikrofluidikstrukturen einer Ebene mit Mikrofluidikstrukturen einer anderen Ebene über Durchgangsöffnungen in mindestens einer Folienlage verbunden sind.
[2] Folienverbund nach [1], der fächerförmig gefaltet ist.
   Gemäß [2] erfolgt das Falten fächerartig, also nach dem Leporello-Prinzip.
[3] Folienverbund nach [1] oder [2], wobei (i) mindestens eine der Folienlagen eine Trägerfolie und auf einer Flächenseite der Trägerfolie eine Lackschicht mit geprägten Vertiefungen aufweist; und/oder wobei (ii) mindestens eine der Folienlagen eine Trägerfolie und auf beiden Flächenseiten der Trägerfolie eine Lackschicht mit geprägten Vertiefungen auf mindestens einer der beiden Flächenseite der Trägerfolie aufweist.

Im Fall von (ii) können Mikrofluidikstrukturen in unterschiedlichen Ebenen dadurch gebildet sein, dass (a) Vertiefungen in zwei benachbarten Folienlagen bedeckt sind und/oder dass (b) Vertiefungen auf beiden Flächenseiten einer Folienlage vorliegen und die Vertiefungen auf beiden Flächenseiten bedeckt sind.

Wenn die Folie fächerartig gefaltet wird, kann ein Folienverbund nach Punkt [3](i) bevorzugt sein, der aus einer Folie gefaltet wird, die abwechselnd einen Abschnitt mit Prägelackschicht auf einer Flächenseite und einen Abschnitt mit Prägelackschicht auf der anderen Flächenseite aufweist und die zwischen den Abschnitten unter Bildung der Folienlagen gefaltet ist. Auf diese Weise kann im gesamten Folienverbund verhindert werden, dass eine Trägerfolie von einer Trägerfolie bedeckt wird.

Es kann Folienverbund nach Punkt [3](ii) bevorzugt sein, wobei auf beiden Flächenseiten der Trägerfolie Vertiefungen vorliegen. In diesem Fall entstehen bei jedem Faltvorgang Mikrofluidikstrukturen unterschiedlicher Ebenen ohne eine dazwischenliegende Trägerfolie. Im Fall von zwei benachbarten Folienlagen können die Flächenseiten, welche die Vertiefungen aufweisen, einander zugewandt sein. Die einander zugewandten Vertiefungen können zumindest teilweise überlappen, so dass eine höhere Mikrofluidikstruktur gebildet wird, die z. B. als Reaktionskammer genutzt werden kann.

[3-1] Vorzugsweise sind in dem Folienverbund nach [3] die Lackschichten von zwei benachbarten Folienlagen durch UV-Härten verbunden.

Durch das UV-Härten werden kovalente Bindungen ausgebildet, was zu einer besonders festen Verbindung der Lagen und zu einer hermetischen Abdeckung der Mikrofluidikstrukturen führt.
[4] Folienverbund nach einem der vorstehenden Punkte, wobei die Folie an Faltkanten gefaltet ist, die durch Laserschneiden der Folien erzeugt wurden, und/oder die Durchgangsöffnungen durch Laserschneiden erzeugt wurden.
[5] Folienverbund nach einem der vorstehenden Punkte, der aus deckungsgleichen Folienlagen, die vollflächig unmittelbar übereinanderliegen, und Faltkanten besteht.
   [5-1] Vorzugsweise ist der Folienverbund nach [5] fächerartig gefaltet.
   [5-2] Vorzugsweise sind in dem Folienverbund nach [5] die Lackschichten von zwei benachbarten Folienlagen durch UV-Härten verbunden.
[6] Folienverbund nach einem der vorstehenden Punkte, wobei mindestens eine der Folienlagen eine Trägerfolie und mindestens eine Lackschicht mit Vertiefungen aufweist, die sich bis zur Trägerfolie erstrecken, so dass die gebildete Mikrofluidikstruktur teilweise von der Trägerfolie bedeckt wird.
   Dadurch wird die Dicke der Lackschicht maximal ausgenutzt. Außerdem wird dadurch ermöglicht, dass eine Mikrofluidikstruktur von unterschiedlichen Materialien bedeckt wird, die beispielsweise unterschiedlich modifiziert sein können. Beispielsweise kann die Trägerfolie funktionelle Gruppen aufweisen, die nach dem Freilegen durch das Prägen mit gewünschten Substanzen gekoppelt werden können. Die funktionellen Gruppe können gezielt an den Stellen erzeugt werden, die später durch das Prägen freigelegt werden.
[7] Folienverbund nach einem der vorstehenden Punkte, der in den Mikrofluidikstrukturen mindestens eine Modifikation aufweist, die unter einer Funktionalisierung, Nanostrukturierung, Hydrophobisierung und Heizmittelfüllung ausgewählt ist.
   Eine Mikrofluidikstruktur kann mehrere dieser Modifikationen aufweisen, wobei die Modifikationen in unterschiedlichen Abschnitten der Mikrofluidikstruktur vorliegen. Durch das Aufeinanderlegen von zwei Vertiefungen beim Falten können sogar in der Draufsicht an ein und derselben Stelle in der Mikrofluidikstruktur zwei getrennte und unterschiedliche Modifikationen vorhanden sein, nämlich eine Modifikation oben und eine unten.
[8] Folienverbund nach einem der vorstehenden Punkte, wobei mindestens eine der Folienlagen eine Trägerfolie und auf mindestens einer Flächenseite der Trägerfolie eine Lackschicht mit geprägten Vertiefungen aufweist, wobei entweder (i) die Vertiefungen in einer Lackschicht der Folienlagen vorliegen und die Lackschicht durch UV-Härten mit der benachbarten Folienlage verbunden ist oder (ii) die Vertiefungen in einer thermoplastischen Lackschicht der Folienlagen vorliegen und die Lackschicht durch ausschließlich thermisches Bonden mit der benachbarten Folienlage verbunden ist.
   Der Folienverbund nach Punkt [8](i) wird in der ersten Ausführungsform beschrieben. Der Folienverbund nach Punkt [8](ii) wird in der zweiten Ausführungsform beschrieben.
[9] Folienverbund nach [1], der fächerförmig gefaltet ist und aus deckungsgleichen Folienlagen, die vollflächig unmittelbar übereinanderliegen, und Faltkanten besteht, wobei die Faltkanten und die Durchgangsöffnungen unter Einsatz eines Lasers erzeugt wurden und wobei die Folienlagen eine Trägerfolie und eine Lackschicht mit Vertiefungen aufweist und die Lackschicht durch UV-Härten mit der benachbarten Folienlage verbunden ist.
[10] Verfahren zum Herstellen eines Folienverbundes nach einem der Punkte [1] bis [9], welches die folgenden Schritte umfasst:
   (a) das Auftragen einer Schicht eines härtbaren Prägelacks auf eine erste Flächenseite einer Trägerfolie unter Bildung einer Prägelackschicht in einem Prägelackabschnitt,
   (b) das Auftragen einer Schicht eines härtbaren Bondinglacks auf die erste Flächenseite der Trägerfolie unter Bildung einer Bondinglackschicht in einem Bondinglackabschnitt,
   (c) das Prägen von Vertiefungen in die in Schritt (a) erhaltene Prägelackschicht mit einem Prägewerkzeug,
   (d) das Teilhärten der in Schritt (c) geprägten Prägelackschicht,
   (e) das Entfernen des Prägewerkzeugs nach Schritt (d),
   (f) das Ausbilden von Durchgangsöffnungen durch die in Schritt (e) erhaltene Struktur von der ersten Flächenseite zu der zweiten Flächenseite der Trägerfolie und das Falten der Struktur zwischen dem Prägelackabschnitt und dem Bondinglackabschnitt unter Bildung von zwei Folienlagen,
   (g) das Aufeinanderlegen der in Schritt (f) erhaltenen zwei Folienlagen unter Bildung eines Verbundes, so dass die Vertiefungen der Prägelackschicht des Prägelackabschnitts der einen Folienlage von der Bondinglackschicht des Bondinglackabschnitts der anderen Folienlage unter Bildung von Mikrofluidikstrukturen abgeschlossen werden,
   (h) das Härten der teilgehärteten Prägelackschicht und der Bondinglackschicht des in Schritt (g) erhaltenen Verbundes unter Ausbildung kovalenter Bindungen zwischen diesen Schichten, wobei der Folienverbund erhalten wird.
[11] Verfahren nach [10], wobei der härtbare Bondinglack ein härtbarer Prägelack ist, der gemäß den Schritten (c) bis (e) geprägt und teilgehärtet wird.
[12] Verfahren zum Herstellen eines Folienverbundes nach einem der Punkte [1] bis [9], welches die folgenden Schritte umfasst:
   (a) das Auftragen einer Schicht eines Prägelacks, der zu einem Polymer radikalisch polymerisierbare Monomere enthält, auf eine erste Flächenseite einer Trägerfolie unter Bildung einer Prägelackschicht in einem Prägelackabschnitt,
   (b) das Auftragen einer Deckschicht auf die erste Flächenseite der Trägerfolie unter Bildung einer Deckschicht in einem Deckschichtabschnitt,
   (c) das Prägen von Vertiefungen in die in Schritt (a) erhaltene Prägelackschicht mit einem Prägewerkzeug,
   (d) das Polymerisieren der in der Prägelackschicht enthaltenen Monomere zu einem Polymer unter Bildung einer thermoplastischen Prägelackschicht auf der Trägerfolie ,
   (e) das Entfernen des Prägewerkzeugs nach Schritt (d),
   (f) das Ausbilden von Durchgangsöffnungen durch die in Schritt (e) erhaltene Struktur von der ersten Flächenseite zu der zweiten Flächenseite der Trägerfolie und das Falten der Struktur zwischen dem Prägelackabschnitt und dem Deckschichtabschnitt unter Bildung von zwei Folienlagen,
   (g) das thermische Aneinander-Bonden der in Schritt (f) erhaltenen zwei Folienlagen, so dass die Vertiefungen der Prägelackschicht des Prägelackabschnitts der einen Folienlage von der Deckschicht des Deckschichtabschnitts der anderen Folienlage unter Bildung von Mikrofluidikstrukturen abgeschlossen werden, wobei der Folienverbund erhalten wird.
[13] Verfahren nach [12], wobei die Deckschicht eine thermoplastische Lackschicht, die gemäß den Schritten (c) bis (e) geprägt und polymerisiert wird.
[14] Verfahren nach einem der Punkte [10] bis [13], wobei das Prägen in einem Rolle-zu-Rolle-Verfahren durchgeführt wird.
[15] Verwendung eines Folienverbundes nach einem der Punkte [1] bis [10] für die Amplifikation von DNA durch Polymerasekettenreaktion oder isothermische Amplifikation.

### Vorteile der Erfindung

Durch Verwendung von dünne Folienlagen mit Mikrofluidikstrukturen erhält man bei gleichbleibender Grundfläche einen größeren verfügbaren Platz und kann mehr Funktionen auf gleicher Fläche unterbringen.

Die Herstellung des erfindungsgemäßen Folienverbundes wird durch das Falten der Folienlagen an Sollfaltlinien erleichtert.

Zur Herstellung der Mikrofluidikstrukturen in dem erfindungsgemäßen Folienverbund kann ein Rolle-zu-Rolle-Prägen der Mikrostrukturen auf großer Fläche und in hohem Durchsatz eingesetzt werden. Diese Mikrostrukturen können gleichzeitig durch Lamination einer Deckschicht mit bekannten Verfahren verschlossen werden. Damit lassen sich geschlossene Kanalnetzwerke erzeugen, die beispielsweise im Einsatz für Biosensoren nötig sind.

Die Mikrofluidikstrukturen sind zwischen der Abdeckung und den Seitenwänden rundum fest und hermetisch verschlossen. Dadurch sind die Mikrofluidikstrukturen mechanisch stabil und unempfindlich gegenüber Druckunterschieden und Belastungen bei der Handhabung. Ein hermetischer Verschluss verhindert auch das Eindringen von Fremdstoffen und Verunreinigungen, die das Analyseergebnis verfälschen könnten, und erlaubt außerdem den Ablauf von temperaturgesteuerten Prozessen (z.B. On-Chip-Amplifikation). Gleichzeitig ergibt sich durch den Aufbau aus Trägerfolien und dünnen Lackschichten eine hohe mechanische Flexibilität, was die Herstellung in kostengünstigen Rollenverfahren und eine problemlose Faltung ermöglicht. Die Mikrofluidikstrukturen sind außerdem ohne den Einsatz eines Klebemittels verschlossen, so dass mögliche Verunreinigungen vermieden werden.

Da die Mikrofluidikstrukturen rundum aus dem gleichen Material bestehen können, ist ein gleichmäßiger und störungsfreier Transport der Flüssigkeiten oder des Analyten möglich.

Das erfindungsgemäße Verfahren ermöglicht ein einfaches UV-Bonden oder thermisches Bonden und kann somit in Hochdurchsatzverfahren eingesetzt werden. Beim UV-Bonden werden kovalente Bindungen erzeugt. Zudem ist beim UV-Bonden kein thermoplastisches Verhalten notwendig und damit keine Einschränkung auf monofunktionale Acrylate erforderlich, so dass sich mehr Freiheiten bei der Materialentwicklung ergeben. Aufgrund der Ausbildung von Vernetzungen beim Härten sind die Kanalmaterialien elastisch, so dass Rissbildungen während der rollenbasierten Herstellung oder in der Anwendung vermieden werden.

Durch die Vielzahl miteinander kommunizierender Mikrofluidikstrukturen verschiedener Größen, Formen und Eigenschaften ist eine Kombination verschiedener Funktionen auf einem einzigen Substrat möglich, so dass Zeit, Kosten und Ressourcen geschont werden. Beispielsweise umfasst eine solche Kombination eine oder mehrere der folgenden Funktionen: eine Probenamplifizierung (bspw. mittels LAMP (Loop-mediated Isothermal Amplification) oder PCR (Polymerase Kettenreaktion)), damit die zu analysierende DNA direkt im Chip amplifiziert werden kann; die Ausblidung hydrophober Verzögerungsstrukturen, damit die Flüssigkeiten, die durch den Chip Fließen, an gewünschten Positionen gestoppt werden können; die Ausbildung strukturierter Sensoroberflächen, um eine größere Oberfläche zu erzeugen und somit eine verbesserte Biofunktionalisierung zu ermöglichen; ein vergrößerte Reaktionskammer; eine vergrößerte Speicherkammer zur Aufnahme zusätzlicher Prozessflüssigkeiten; die Ausgestaltung von Durchgangsöffnungen zwischen den Folienlagen derart, dass sie eine Flüssigkeit kapillargetrieben von Lage zu Lage ziehen und die Erzeugung von Heizleiterstrukturen für die bei der Probenamplifizierung nötige Temperierung, welche durch kapillargetriebene Befüllung der mikrofluidische Strukturen mit metallischen Tinten und nachfolgender Sinterung erfolgen (Heizmittelfüllung).

### Beschreibung der Figuren

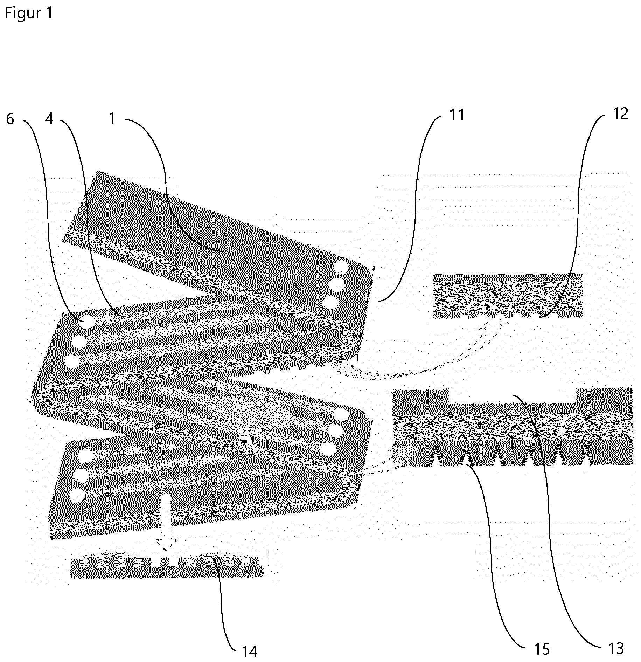

Figur 1 veranschaulicht das Prinzip der Herstellung des erfindungsgemäßen Folienverbundes am Beispiel eines Folienverbundes zur Verwendung in der Amplifikation von DNA. Die Temperatur für die isothermische PCR wird über die heizmittelgefüllten Mikrofluidikstrukturen (15) unter der PCR-Kammer (13) eingestellt. Die mikrostrukturierte Oberfläche (14) kann eine Biofunktionalisierung (z. B. Anbindung von DNA und/oder Protein) aufweisen. Das hydrophobe Ventil (12) dient der selektiven Wechselwirkung der Oberfläche mit Substanzen und beispielsweise dem selektiven Durchgang zu benachbarten Mikrofluidikstrukturen.

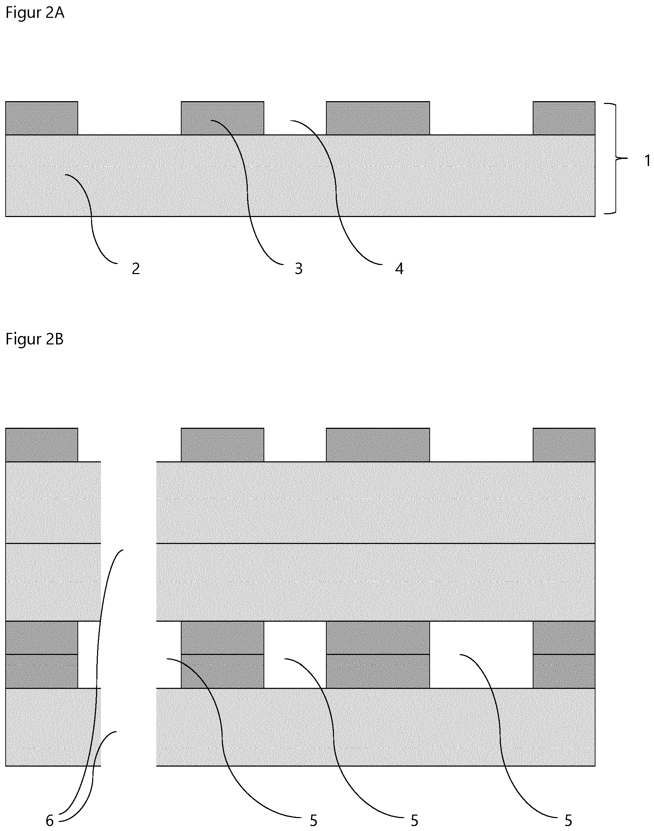

Figur 2A zeigt schematisch einen Querschnitt durch eine Folienlage (1). Auf der Trägerfolie (2) ist einseitig eine Prägelackschicht (3) mit Vertiefungen (4) einer Tiefe von 100 % aufgetragen.
Figur 2B zeigt einen Querschnitt eines erfindungsgemäßen Folienverbundes aus Folienlagen, die einseitig eine Prägelackschicht aufweisen (vgl. Figur 2A). Die Faltkante ist nicht gezeigt. Die Vertiefungen sind durch die benachbarten Folienlagen unter Bildung von im Querschnitt geschlossenen Mikrofluidikstrukturen (5) abgedeckt. Die Durchgangsöffnungen (6) verbinden Mikrofluidikstrukturen (5) unterschiedlicher Ebenen.

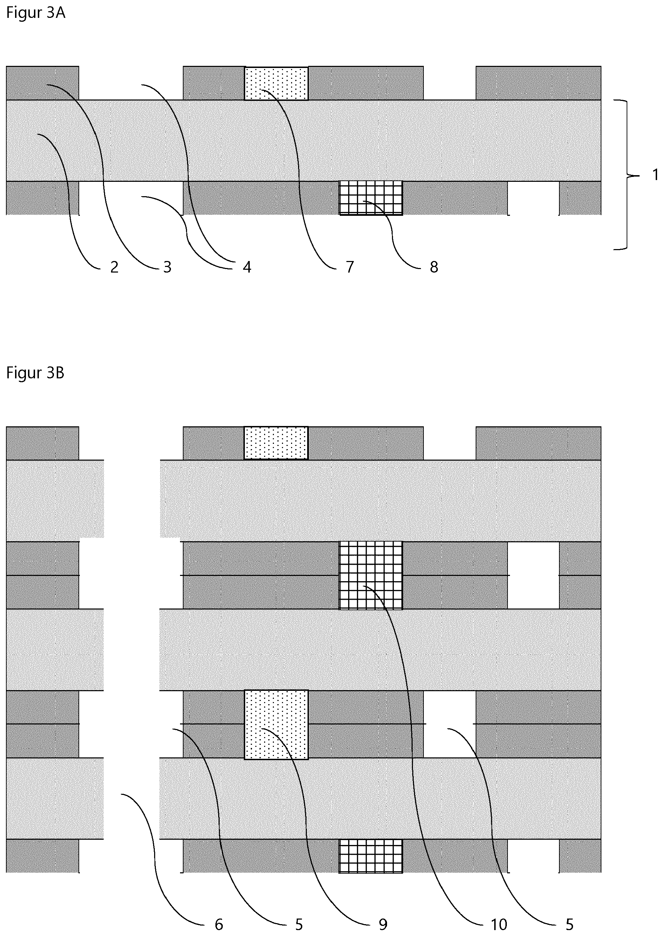

Figur 3A zeigt schematisch einen Querschnitt durch eine Folienlage (1). Auf der Trägerfolie (2) ist zweiseitig eine Prägelackschicht (3) mit Vertiefungen (4) einer Tiefe von 100 % aufgetragen. Die Vertiefungen (4) können unterschiedliche Funktionalitäten aufweisen. Beispielsweise kann eine Vertiefung (4) Heizmittel (7) oder Nanostrukturen (8) enthalten.
Figur 3B zeigt einen Querschnitt eines erfindungsgemäßen Folienverbundes aus Folienlagen, die zweiseitig eine Prägelackschicht aufweisen (vgl. Figur 3A). Die Faltkante ist nicht gezeigt. Die Vertiefungen sind durch die benachbarten Folienlagen unter Bildung von im Querschnitt geschlossenen Mikrofluidikstrukturen (5) abgedeckt. Die Durchgangsöffnungen (6) verbinden Mikrofluidikstrukturen (5) unterschiedlicher Ebenen. Die Mikrofluidikstrukturen (5) können unterschiedliche Funktionalitäten aufweisen. Beispielsweise kann eine Mikrofluidikstruktur (5) Heizmittel (9) oder Nanostrukturen (10) enthalten.

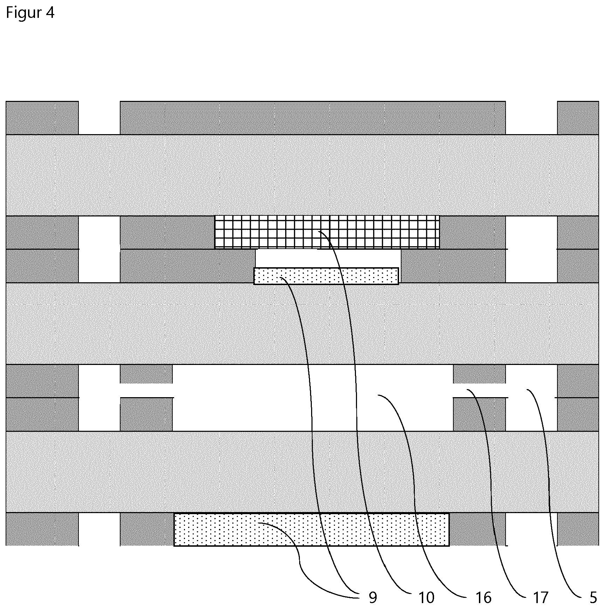

Figur 4 zeigt einen Querschnitt eines erfindungsgemäßen Folienverbundes aus Folienlagen, die zweiseitig eine Prägelackschicht aufweisen. Die Faltkante und die Durchgangsöffnungen sind nicht gezeigt. Die Vertiefungen sind durch die benachbarten Folienlagen unter Bildung vom im Querschnitt geschlossenen Mikrofluidikstrukturen (5) abgedeckt. Eine Mikrofluidikstruktur (5) kann im Querschnitt unterschiedliche Funktionalitäten aufweisen. Beispielsweise kann eine Mikrofluidikstruktur (5) Heizmittel (9) und Nanostrukturen (10) enthalten. Eine Mikrofluidikstruktur (5) kann durch Bildung aus breiten Vertiefungen (4) (vgl. Fig. 2A, 2B und 3A) einer Tiefe von 100 % einen größeren Hohlraum (16) ausbilden, der als Reaktionskammer oder Speicherkammer genutzt werden kann. Der Hohlraum (16) ist über eine Verbindungsvertiefung (17) mit einer Mikrofluidikstruktur (5) verbunden.

In den Figuren haben die Mikrofluidikstrukturen eine rechteckige Form und zumeist eine Tiefe von 100 % der Schichtdicke des Prägelackschicht. Es ist zu beachten, dass die Mikrofluidikstrukturen jede andere Form haben können, die durch Prägen erzeugbar ist, beispielsweise ein zur Trägerfolie hin zulaufende dreieckige Form oder abgerundete Form. Außerdem kann die Tiefe auch weniger als 100 % betragen, so dass die Mikrofluidikstruktur nicht teilweise von der Trägerfolie abgedeckt wird.

### Ausführungsformen der Erfindung

Der erfindungsgemäße Folienverbund ist aus einer einzelnen Folie gefaltet. Das heißt, der Folienverbund besteht aus einer Folie, die gefaltet ist. Folglich sind andere Komponenten, beispielsweise Klebstoffe, oder andere Schichten oder Lagen zwischen den Folienlagen ausgeschlossen, wohingegen in den Mikrofluidikstrukturen zusätzliche Komponenten, wie Nanostrukturen, Heizmittel, Funktionalisierungen und dergleichen, nicht ausgeschlossen sind. Die Folie ist mehrfach gefaltet und bildet mehrere Folienlagen und dazwischenliegende Faltkanten.

In einer Ausführungsform ist der Folienverbund so ausgebildet, dass die geschichteten Folienlagen vollflächig übereinanderliegen, das heißt, dass sie auch unmittelbar nach einer Faltkante verbunden sind. Der Folienverbund besteht also aus deckungsgleichen Folienlagen, die vollflächig unmittelbar übereinanderliegen, und Faltkanten. Die Folienlagen haben gleiche Länge und Breite und Flächenform, vorzugsweise sind sie Rechtecke gleicher Größe, und die Faltkanten befinden sich in vertikaler Richtung des Folienverbundes abwechselnd an einem Ende und an dem dazu gegenüberliegenden Ende der Folienlagen, vorzugsweise an den gegenüberliegenden Flächenseiten der rechteckigen Folienlagen.

Der Folienverbund ist somit vorzugsweise nach dem Leporello-Prinzip gefaltet, also fächerartig wie beispielsweise ein Stapel Endlospapier. Dadurch liegen auf mindestens einer Flächenseite einer Folienlage keine Bereiche vor, die nicht mit einer benachbarten Folienlage verbunden sind. Auf diese Weise wird die Oberfläche der Folienlagen maximal genutzt.

Alternativ oder zusätzlich zu der Faltung nach dem Leporello-Prinzip ist natürlich jede Art der Faltung möglich. Beispielsweise kann eine Folie, die zwei Faltkanten und damit eine mittlere Folienlage sowie eine erste und eine zweite äußere Folienlage aufweist, so gefaltet werden, dass die erste äußere Folienlage über die mittlere Folienlage gefaltet wird und die zweite äußere Folienlage anschließend über die erste Folienlage gefaltet wird.

Vorliegend bedeutet im Zusammenhang mit Folienlagen der Ausdruck "geschichtete Folienlagen", dass in dem Folienverbund jede Folienlage unmittelbar mit einer anderen Folienlage verbunden ist. Vorliegend bedeutet der Ausdruck "Schichtungsrichtung" die z-Richtung senkrecht zur x-y-Ebene einer flächigen Folienlage und gibt ein Position über oder unter der Folienlage an. Im Zusammenhang mit Folienlagen bedeutet der Ausdruck "in Schichtungsrichtung benachbart", dass eine erste Folienlage unmittelbar mit einer zweiten Folienlage verbunden ist und in z-Richtung über oder unter der zweiten Folienlage liegt.

Vorliegend bedeutet der Ausdruck "in unterschiedlichen Ebenen" unterschiedliche Höhe in z-Richtung. Eine unterschiedliche Ebene beispielsweise von Mikrofluidikstrukturen kann sich dadurch ergeben, dass die Mikrofluidikstrukturen innerhalb eines Folienverbundes in verschiedenen Folienlagen oder auf Unter- und Oberseite derselben Folienlage vorliegen.

Eine Folienlage ist dadurch definiert, dass sie von einer anderen Folienlage durch Faltung der Folie, insbesondere durch eine Faltkante getrennt ist.

Die Folie und damit auch jede Folienlage ist flächig mit Ausmaßen in x- und y-Richtung, welche die Kontaktfläche mit den in Schichtungsrichtung benachbarten Folienlagen definieren, und z-Richtung, welche der Foliendicke bzw. Folienlagendicke entspricht. Die Ausmaße in x- und y-Richtung sind jeweils mindestens das Zehnfache, vorzugsweise mindestens das Zwanzigfache, stärker bevorzugt mindestens das Fünfzigfache des Ausmaßes in z-Richtung. Die Folienlage ist für sich genommen eine dünne Folie.

Wenn in der vorliegenden Erfindung für die Bezeichnung von Elementen oder Strukturen die Einzahl verwendet wird, beispielsweise "eine Mikrofluidikstruktur" oder "die Mikrofluidikstruktur", soll damit eine Mehrzahl dieser Elemente oder Strukturen ausdrücklich nicht ausgeschlossen sein, so dass die beispielhaften Bezeichnungen gleichbedeutend sind mit "mindestens eine Mikrofluidikstruktur" bzw. "die mindestens eine Mikrofluidikstruktur", sofern nichts Gegenteiliges angegeben ist.

### Die Folie

Die Folie besteht aus einer Trägerfolie und optional auf einer oder auf beiden ihrer Flächenseiten weiteren Schichten, beispielsweise einer Prägelackschicht oder Bondingschicht.

Aus der Folie werden die Folienlagen für den Folienverbund gefaltet.

### Trägerfolie

Die Trägerfolie ist eine flächige Polymerfolie mit einer Fläche in x-y-Richtung und einer Dicke in z-Richtung.

Die Trägerfolie weist vorzugsweise eine Dicke von 5 bis 2000 µm, bevorzugt 10 bis 1000 µm, besonders bevorzugt 20 bis 500 µm auf. Sie ist vorzugsweise eine Kunststofffolie aus PI, PP, MOPP, PE, PPS, PEEK, PEK, PEI, PSU, PAEK, LCP, PEN, PBT, PET, PA, PC, COC (Cycloolefincopolymer), POM, ABS, PVC, PTFE, ETFE (Ethylentetrafluorethylen), PTFE (Polytetrafluorethylen), PVF (Polyvinylfluorid), PVDF (Polyvinylidenfluorid), und EFEP (EthylenTetrafluorethylen-Hexafluorpropylen-Fluorterpolymer), COP (Cycloolefin polymer), PS (Polystyrol).

Die Oberfläche der Trägerfolie kann Funktionalisierungen aufweisen, beispielsweise Hydroxy-, Thio- oder Amingruppen. Alternativ dazu können die Funktionalisierungen mit Schutzgruppen geschützt sein, welche nach der Prägung und Polymerisierung des Ausgangsmaterials der Prägelackschicht entfernt werden. Der Schutz der Funktionalisierungen kann auch zur Hydrophobierung der Oberfläche betragen, so dass die Materialien der Trägerfolie und der Prägelackschicht kompatibler werden und damit die nichtkovalente Anhaftung verstärkt wird.

Die Funktionalisierungen können zur kovalenten Anbindung der Polymere der Prägelackschicht dienen.

Die Funktionalisierungen können auch dazu dienen, gewünschte Verbindungen für beispielsweise diagnostische Zwecke kovalent anzubinden. Solche Verbindungen können Biomoleküle sein, beispielsweise Nukleinsäuren, Proteine, wie Antikörper, Lipide oder Kohlenhydrate sein. Außerdem können über diese Funktionalisierungen Moleküle mit zwei reaktiven Gruppen angebunden werden, wobei eine reaktive Gruppe mit der Funktionalisierung der Trägerfolie eine kovalente Bindung eingeht und die andere reaktive Gruppe mit einer Zielsubstanz reagieren kann.

In der Regel befinden sich mögliche Funktionalisierungen jedoch im oder auf dem Prägelack.

### Die Prägelackschicht

Die Lackschicht, die auf der Trägerfolie vorliegt und die geprägten Vertiefungen aufweist, wird als Prägelackschicht bezeichnet. Sie hat eine Dicke, also ein Ausmaß senkrecht zur Kontaktfläche mit der Flächenseite der Trägerfolie, von vorzugsweise 10 nm bis 1000 µm, stärker bevorzugt 50 nm bis 500 µm,noch stärker bevorzugt 100 nm bis 100 µm. In einzelnen Ausführungsformen kann die Dicke 100 nm bis 50 µm sein.

Die Tiefe der Prägung hängt von der Dicke der Lackschicht und der Tiefe der Stempelstrukturen ab. Die Prägungstiefe kann 1 bis weniger als 100 % oder sogar 1 bis 100 % der Dicke der Prägelackschicht sein. Vorzugsweise ist sie 50 bis 100 %, stärker bevorzugt 50 bis weniger als 100 %, noch stärker bevorzugt 80 bis 95 % der Dicke der Prägelackschicht. Die Kombination der Dicke der Prägelackschicht (in µm) und der Prägungstiefe (in %) kann vorzugsweise 0,1 bis 500 µm und 10 bis 100 %, stärker bevorzugt 0,1 bis 100 µm und 70 bis 100 % oder 0,1 bis 50 µm und 30 bis 100 % sein. In einzelnen Ausführungsformen kann die Prägungstiefe 50 bis 200 µm sein und dabei 50 bis 100 % der Dicke der Prägelackschicht sein.

In der Regel beträgt die Prägungstiefe weniger als 100 % der Dicke der Prägelackschicht, so dass die Mikrofluidikstruktur auf drei Seiten (zwei Wände und Boden) das gleiche Material aufweist. Wenn die Abdeckung der Mikrofluidikstruktur aus dem gleichen Material wie die Prägelackschicht besteht, wird die Mikrofluidikstruktur rundum durch das gleiche Material gebildet.

In einer besonderen Ausführungsform kann die Prägungstiefe 100 % der Dicke der Prägelackschicht sein, das heißt, es ist innerhalb der Prägung ein Bereich vorhanden, in dem kein Lackschichtmaterial auf dem Träger verbleibt. In diesem Fall ist die Prägelackschicht keine durchgehende, sondern eine unterbrochene Schicht (vgl. Figuren 2 bis 4). Generell machen diese Unterbrechungen der Prägelackschicht an der Kontaktfläche zur Trägerfolie vorzugsweise 1 bis 90 %, stärker bevorzugt 5 bis 70 %, noch stärker bevorzugt 10 bis 60 % der Gesamtfläche der Prägelackschicht aus.

Wenn die Prägungstiefe 100 % der Dicke der Prägelackschicht ist, befindet sich in dem geprägten Bereich eine von Prägelackschicht freie, also zugängliche Oberfläche der Trägerfolie , die sich von der Oberfläche der Vertiefungen in der Prägelackschicht unterscheiden kann. Diese Unterschiede können beispielsweise darin bestehen, dass die Oberfläche der Trägerfolie Funktionalisierungen aufweist, beispielsweise Hydroxy-, Thio-oder Amingruppen. werden. Bei einer Prägungstiefe von 100 % erstrecken sich die Hohlräume von der Unterseite der Deckschicht bis zur Oberfläche der Trägerfolie , wobei Dickenbereiche der Prägelackschicht die Seitenwände der Hohlräume bilden.

Die in dem geprägten Bereich der Prägelackschicht freiliegenden Funktionalisierungen auf der Oberfläche der Trägerfolie können wie weiter oben beschrieben dazu genutzt werden, gewünschte Verbindungen vorzugsweise kovalent an die Trägerfolie zu binden. Auf diese Weise können in die geprägten Bereiche der Prägelackschicht gezielt Verbindungen eingeführt werden, ohne dass eine Anbindungsmöglichkeit an die Prägelackschicht bestehen muss. Dadurch besteht eine größere Freiheit bei der Auswahl der Ausgangsmaterialien der Prägelackschicht.

### Mikrofluidikstrukturen

Der erfindungsgemäße Folienverbund enthält Mikrofluidikstrukturen, die Hohlräume sind, die dadurch entstehen, dass eine Folienlage, die Vertiefungen aufweist, durch eine benachbarte Folienlage bedeckt wird. Sie sind also sandwichartig zwischen zwei Folien angeordnet.

Die Mikrofluidikstrukturen können aus Vertiefungen in einer Prägelackschicht auf einer Trägerfolie gebildet werden, die von der benachbarten Folienlage, also einer Deckschicht bedeckt sind. Die Prägelackschicht ist auf der der Deckschicht zugewandten Flächenseite geprägt. Die Deckschicht befindet sich unmittelbar auf der Lackschicht. Stärker bevorzugt wird die Prägeseite der Lackschicht mit Ausnahme der Vertiefungen der Prägung vollflächig und unmittelbar von der Deckschicht abgedeckt. In den Vertiefungen der Prägung befindet sich vorzugsweise weder Lackschichtmaterial noch Deckschichtmaterial, so dass Hohlräume entstehen, die fluidgefüllt sein können. Der Ausdruck "unmittelbar" bedeutet, dass kein weiteres Material, beispielsweise ein Kleber, zwischen der Lackschicht und der Deckschicht vorhanden ist.

Die Mikrofluidikstruktur hat Ausmaße im Mikrometermaßstab in z-Richtung und entweder in x-Richtung oder y-Richtung, wobei die x-y-Ebene durch die Flächen der Folienlagen definiert ist. Die Mikrofluidikstruktur weist beispielsweise eine Tiefe (in z-Richtung) und Breite (x- oder y-Richtung) von jeweils 0,1 bis 200 µm, bevorzugt 1 bis 50 µm auf. Die Länge der Mikrofluidikstruktur ist nicht eingeschränkt. Die Mikrofluidikstruktur ist für die Aufnahme und den Transport von Fluiden geeignet, also von Gasen oder Flüssigkeiten. Ein Fluid ist bei 21°C eine Flüssigkeit oder ein Gas, insbesondere Luft. Die Mikrofluidikstruktur können auch mit einem festen Material gefüllt sein, das sich sowohl vom Material der Prägelackschicht als auch vom Material der Deckschicht unterscheidet. Ein solchen Material ist beispielsweise ein Heizmittel.

Bei 100 % Prägungstiefe werden die Hohlräume zwischen der Trägerfolie und der benachbarten Folienlage, bei einer geringeren Prägungstiefe zwischen der Prägelackschicht und der benachbarten Folienlage gebildet.

Eine Mikrofluidikstruktur ist rundum fest verschlossen. Der Ausdruck "rundum" in Bezug auf die Abdeckung bedeutet hier, dass eine Mikrofluidikstruktur an jedem Punkt in z-Richtung, also in der Richtung senkrecht zur Substratfolienebene, und in x-Richtung oder y-Richtung abgeschlossen ist. Mit Ausnahme der Bereiche mit Durchgangsöffnungen oder Verbindungsvertiefungen gibt es somit an jedem Punkt einer Mikrofluidikstruktur eine Querschnittsebene in y-Richtung, in der die Mikrofluidikstruktur rundum abgeschlossen ist. Die Mikrofluidikstrukturen weisen mindestens eine Öffnung, vorzugsweise mindestens eine Einlassöffnung und mindestens eine Auslassöffnung, vorzugsweise an einem ihrer Enden in der x-y-Ebene auf.

### Das Prägen

Die Folienlagen weisen geprägte Vertiefungen auf. Hierin bezeichnet "prägen" einen Vorgang, bei dem ein ausreichend weiches Substrat mit einem Prägewerkzeug, das die Negativform der gewünschten Vertiefung aufweist, derart kontaktiert wird, dass in dem Substrat die gewünschte Vertiefung erzeugt wird.

Das Substrat kann ein nicht oder nicht vollständig polymerisiertes bzw. gehärtetes Polymerausgangsmaterial sein, das nach dem Prägen polymerisiert bzw. gehärtet wird. Das Substrat kann aber auch ein erweichtes Polymer sein, das nach dem Prägen erstarrt wird. Beispielsweise kann das feste Polymer thermisch aufgeweicht, geprägt und dann durch Abkühlen wieder verfestigt werden. Ebenso kann das feste Polymer in einem Extruder thermisch geschmolzen und nach dem Extrudierun und Prägen durch Abkühlen wieder verfestigt werden. Das Prägewerkzeug, das die Negativform der gewünschten Vertiefung aufweist, kann beispielsweise ein Prägestempel oder eine Walze sein.

Ein Beispiel eines Prägeverfahrens ist die Ultraviolett-Prägelithografie (Englisch: ultraviolett nanoimprint lithography, kurz UV-NIL). Dabei wird ein flüssiger Prägelack im kalten Zustand auf ein Polymersubstrat aufgetragen. Ein nanostrukturierter Stempel mit dem invertierten Profil der Vertiefungen wird als Negativform eingesetzt und in den Prägelack gepresst, in dem dann die gewünschte Struktur als Positivform eingeprägt ist. Durch Bestrahlung mit UV-Licht polymerisiert der Prägelack und wird fest. Nach Trennen des Stempels von dem geprägten Muster wird das Profil des Stempels in invertierter Form repliziert. Dieses Verfahren lässt sich bei Verwendung eines zylindrischen Stempels in einem kontinuierlichen Rolle-zu-Rolle-Verfahren umsetzen. Damit können sehr große Flächen in kurzer Zeit strukturiert werden.

Beispiele für Prägevorgänge werden weiter unten bei der Beschreibung des Folienverbundes der ersten und zweiten Ausführungsform erläutert.

Ein weiteres Beispiel der Ausbildung von geprägten Vertiefungen in einem erfindungsgemäßen Folienverbund ist das Extrudieren eines Polymers und das Prägen mit einer Walze.

In jedem Fall kommt es beim Prägen zu einem Kontakt und dadurch zu Wechselwirkungen zwischen dem ausreichend weichen Substrat und dem festen Prägewerkzeug. Diese Wechselwirkungen sind beispielsweise hydrophobe oder ionische Wechselwirkungen. Da das Substrat nicht starr ist, sind die darin enthaltenen Moleküle beweglich. Aufgrund der Wechselwirkungen mit dem Prägewerkzeug kommt es in jedem Fall zu einer gewissen Orientierung der Substratmoleküle und damit zu einer Änderung der Substratoberfläche, die sich am geprägten und festen Substrat nachweisen lässt. Beispielsweise unterscheiden sich die geprägten Oberflächen von den nicht geprägten Oberflächen des Substrats in ihrer Benetzbarkeit mit Wasser.

Durch diese Änderung der Struktur und damit auch der Eigenschaften der geprägten Substratoberfläche kann eine geprägte Vertiefung auch von einer Vertiefung unterschieden werden, die beispielsweise mithilfe eines Lasers erzeugt wurde. Das Substrat einer Laserbehandlung ist nicht weich, sondern starr, so dass eine Orientierung der Substratmoleküle und damit eine Änderung der Oberflächeneigenschaften ausgeschlossen ist. Außerdem kommt es im Gegensatz zu einem Prägevorgang bei einer Laserbebehandlung aufgrund ihrer Natur als subtraktives Verfahren und aufgrund des hohen Energieeintrags unvermeidlich zu Zerstörungen und Modifikationen von Oberflächenmolekülen des Substrats.

### Folienverbund und seine Herstellung gemäß der ersten Ausführungsform

Die Herstellung von Mikrofluidikstrukturen gemäß erster Ausführungsform ist in der am 2. Juni 2020 eingereichten deutschen Patentanmeldung mit dem Aktenzeichen 10 2020 114 621.3 beschrieben.

Das Verfahren zum Herstellen eines Folienverbundes gemäß der ersten Ausführungsform umfasst die folgenden Schritte:
(a) das Auftragen einer Schicht eines härtbaren Prägelacks auf eine erste Flächenseite einer Trägerfolie unter Bildung einer Prägelackschicht in einem Prägelackabschnitt,
(b) das Auftragen einer Schicht eines härtbaren Bondinglacks auf die erste Flächenseite der Trägerfolie unter Bildung einer Bondinglackschicht in einem Bondinglackabschnitt,
(c) das Prägen von Vertiefungen in die in Schritt (a) erhaltene Prägelackschicht mit einem Prägewerkzeug,
(d) das Teilhärten der in Schritt (c) geprägten Prägelackschicht,
(e) das Entfernen des Prägewerkzeugs nach Schritt (d),
(f) das Ausbilden von Durchgangsöffnungen durch die in Schritt (e) erhaltene Struktur von der ersten Flächenseite zu der zweiten Flächenseite der Trägerfolie und das Falten der Struktur zwischen dem Prägelackabschnitt und dem Bondinglackabschnitt unter Bildung von zwei Folienlagen,
(g) das Aufeinanderlegen der in Schritt (f) erhaltenen zwei Folienlagen unter Bildung eines Verbundes, so dass die Vertiefungen der Prägelackschicht des Prägelackabschnitts der einen Folienlage von der Bondinglackschicht des Bondinglackabschnitts der anderen Folienlage unter Bildung von Mikrofluidikstrukturen abgeschlossen werden,
(h) das Härten der teilgehärteten Prägelackschicht und der Bondinglackschicht des in Schritt (g) erhaltenen Verbundes unter Ausbildung kovalenter Bindungen zwischen diesen Schichten, wobei der Folienverbund erhalten wird.

Vorzugsweise ist der härtbare Bondinglack ein härtbarer Prägelack, der gemäß den Schritten (c) bis (e) geprägt und teilgehärtet wird.

Ein bevorzugter erfindungsgemäßer Folienverbund ist gemäß dem Verfahren der ersten Ausführungsform herstellbar.

In dieser Ausführungsform sind die Mikrofluidikstrukturen fest verschlossen. Der Ausdruck "fest" bedeutet hier, dass die Abdeckung einer Mikrofluidikstruktur nicht zerstörungsfrei, beispielweise durch Erwärmen, abgelöst werden kann. Grundlage für die feste Abdeckung ist in der vorliegenden Erfindung die kovalente Bindung zwischen den Ausgangsstrukturen. Der feste Verschluss der Mikrofluidikstrukturen wird dadurch erzielt, dass die Oberflächen der Elemente miteinander chemisch verbunden, also kovalent gebunden sind. Dadurch entsteht ein nicht mehr in die Einzelteile auftrennbarer Schichtaufbau mit einer eingebetteten Mikrofluidikstruktur. Ein hermetischer Verschluss verhindert auch das Eindringen von Fremdstoffen und Verunreinigungen, die das Analyseergebnis verfälschen könnten, und erlaubt außerdem den Ablauf von temperaturgesteuerten Prozessen (z.B. On-Chip-Amplifikation).

Die Mikrofluidikstrukturen sind ausschließlich mit gehärtetem Prägelack und gehärtetem Bondinglack verschlossen. Das heißt, der Boden, die Seitenwände und die Abdeckung bestehen ausschließlich aus diesen gehärteten Lacken, mit der Ausnahme, dass bei einer Prägung einer Tiefe von 100 % der Boden die Trägerfolie ist. Diese Ausformung schließt andere Substanzen aus, beispielsweise ein Klebemittel zwischen Bodenteil und Deckteil.

Der härtbare Prägelack und der härtbare Bondinglack können gleich oder verschieden voneinander sein. Die in dieser Ausführungsform verwendeten Begriffe "Lack" und "Lackschicht" beziehen sich sowohl auf den härtbaren Prägelack als auch unabhängig davon auf den härtbaren Bondinglack bzw. den daraus gebildeten Schichten, solange nichts Gegenteiliges angegeben ist. Die Lackschicht umfasst einen oder besteht aus einem Lack.

Der Lack ist härtbar, beispielsweise thermisch oder durch Einsatz von UV-Strahlen oder Elektronenstrahlen. Die Härtung mit UV-Strahlen ist bevorzugt. Der Lack enthält vorzugsweise mindestens eine Verbindung mit polymerisierbaren C-C-Doppelbindungen. Die Verbindung kann aus der Gruppe ausgewählt sein, die aus Acrylaten, Methacrylaten, Vinylethern, Allylethern, Propenylethers, Alkenen, Dienen, ungesättigten Estern - insbesondere Polyitaconaten , Allyltriazinen, Allylisocyanates und N-Vinylamiden besteht. (Meth)acrylate sind bevorzugt. Urethanacrylate sind bevorzugt. Zusätzlich zu der Verbindung mit polymerisierbaren C-C-Doppelbindungen kann der Lack auch thiolhaltige Verbindungen enthalten. Die Polyitaconate sind ein Spezialfall der ungesättigten Ester - aber dennoch sehr erwähnenswert.

Geeignete UV-vernetzbare Lacke basieren beispielsweise auf Polyethylenglykol-Diacrylaten (PEGDA), ggf. mit 1-10 Massenprozent an höherfunktionalen Acrylaten oder Lacksysteme auf Basis von hochvernetzenden multifunktionellen Polyether-, Polyester- oder Polyurethan-Acrylatsystemen. Bevorzugt sind Polyethylenglykoldiacrylate, ethoxylierte (ganz wichtig!) Trimethylolpropantriacrylate, Acryloylmorpholin, ethoxylierte Pentaerythritoltetraacrylat, Hexandioldiacrylat und trifunktionelles Urethan-Acrylat-Oligomer. Weitere Beispiele sind Triglyzeroldiacrylat/Glycerol-1,3-diglycerolatdiacrylate und polyethoxylierts Pentaerythritoldiacrylat und -triacrylat. Die nicht acrylierten der vier OH-Gruppen von Pentaerythrit können mit den wässrigen Lösungen in der Mikrofluidikstruktur wechselwirken oder für Funktionalisierungen verwendet werden.

UV-vernetzbare Acrylatlacke sind besonders bevorzugt. Sie sind gut in einem Rolle-zu-Rolle-Verfahren zu verarbeiten, da sie keine Vorprozesse (Prebake wie z. B. SU8) benötigen und sehr schnell (in weniger als 1 s) vernetzen. Sie sind außerdem im Hinblick auf ihre Oberflächenenergie und damit ihre Benetzungseigenschaften, welche in der kapillargetriebenen Mikrofluidik eine wesentliche Rolle spielt, sehr gut einstellbar.

Der Lack kann 0,05 bis 5 Massenprozent Photoinitiatoren enthalten, die die Vernetzung unter UV-Strahlung bewirken, beispielsweise Photoinitiatoren auf Basis von Acylphosphinoxiden, oder oligomerer polyfunktionaler Alphahydroxyketone oder monomerer Alphahydroxyketone.

Der Prägelack und der Bondinglack können unabhängig voneinander Additive enthalten. Beispiele solcher Additive sind oberflächenaktive Antihaftadditive und Reaktiwerdünner.

Um ein Anhaften des Prägelacks an das Prägewerkzeug zu verringern und das Entfernen des Prägewerkzeugs nach dem Prägen zu erleichtern, kann dem als Ausgangsmaterial eingesetzten Prägelack ein oberflächenaktives Antihaftadditiv zugesetzt werden, welches silikonhaltig oder fluorhaltig sein kann. Insbesondere ist das Additiv mindestens ein aus der Gruppe ausgewähltes Mitglied, die silikonhaltige oder fluorhaltige Additive umfasst. Diese Additive tragen zur Verringerung der Adhäsion und zur Erleichterung der Ablösung des Prägelacks von dem Prägewerkzeug bei. Die genannten Antihaftadditive können nach der Durchführung des erfindungsgemäßen Verfahrens zumindest teilweise in der geprägten Strukturschicht enthalten sein, beispielsweise in einer Menge von 0,1 bis 3 Gew.-%.

In dem Prägelack und/oder dem Bondinglack kann ein Reaktivverdünner enthalten sein. Ein Reaktivverdünner enthält niedermolekulare Verbindungen, um die Viskosität des Lacks einzustellen.

Das Lackschichtmaterial wird auf eine Substratfolie aufgetragen. Da das Lackschichtmaterial nicht gehärtet, also nicht polymerisiert ist, weist es eine geringe Viskosität auf, so dass es z. B. durch Streichen oder durch Gießen aufgetragen werden kann. Vorzugsweise wird es durch Gravurdruck oder Schlitzdüsenbeschichtung aufgetragen.

Die Viskosität wird so eingestellt, dass das Material leicht aufgetragen werden kann. Andererseits muss es so viskos sein, dass die beim Prägen gebildete Struktur erhalten bleibt. Um beide Erfordernisse zu erfüllen, kann das auf den Träger aufgebrachte Material zunächst moderat bestrahlt werden, so dass ein Teilhärten und damit ein Erhöhen der Viskosität erfolgt. Der Lack kann nach dem Teilhärten bereits fest sein, er weist aber nicht-abreagierte reaktive Gruppen auf. Die gewünschte niedrige Viskosität weist das Lackschichtmaterial vorzugsweise bei Raumtemperatur auf. Dadurch kann das Auftragen des Lackschichtmaterials bei niedrigen Temperaturen, wie Raumtemperatur, erfolgen.

Die Prägelackschicht hat eine Dicke, also ein Ausmaß senkrecht zur Kontaktfläche mit der Substratfolie, von vorzugsweise 100 nm bis 1000 µm, stärker bevorzugt 1 µm bis 100 µm. In einzelnen Ausführungsformen kann die Dicke 1 µm bis 50 µm sein.

Die Bondinglackschicht ist vorzugsweise dünner als die Prägelackschicht und hat vorzugsweise eine Dicke von weniger als 5 µm, stärker bevorzugt weniger als 1 µm. Eine bevorzugte Kombination ist eine Prägelackschicht mit einer Dicke von 1 µm bis 100 µm und eine Bondinglackschicht mit einer Dicke von weniger als 1 µm.

Nachfolgend werden die Schritte des Verfahrens der ersten Ausführungsform eingehend beschrieben.

### Schritte (a) und (b):

Das Auftragen einer Schicht eines härtbaren Prägelacks und einer Schicht eines härtbaren Bondinglacks auf dieselbe Flächenseite der Trägerfolie wird beispielsweise mit einem Rolle-zu-Rolle-Verfahren durchgeführt, bei dem das Auftragen der Lackschicht auf die Substratfolie beispielsweise mittels einer Schlitzdüse oder durch Tiefdruck mit einer Gravurwalze erfolgt. Wenn nach dem Leporello-Prinzip gefaltet wird, können die Prägelackschicht und die Bondinglackschicht in unterschiedlichen Abschnitten derselben Flächenseite der Trägerfolie gebildet werden, so dass sie später durch Umlegen an einer zwischen den beiden Abschnitten befindlichen Faltkante aufeinandergelegt werden können. Wenn nach einem anderen Prinzip gefaltet wird, beispielsweise durch Umlegen von zwei äußeren Folienlagen auf eine mittlere Folienlage, können die Prägelackschicht und die Bondinglackschicht auf entgegengesetzten Flächenseiten, also vorderseitig und rückseitig, der Trägerfolie gebildet werden.

Die Dicke der Bondinglackschicht ist vorzugsweise gering, um in Schritt (h) eine schnellere Härtung zu ermöglichen und beim Bilden des Verbundes in Schritt (g) ein Verblocken der Vertiefungen zu verhindern. Wenn die Bondinglackschicht zu dick ist, kann in Schritt (g) das noch flüssige Material in die Vertiefungen der Prägelackschicht einfließen oder gedrückt werden, noch bevor die Oberflächen und Schichten chemisch gehärtet, also kovalent verbunden sind. Beispielsweise hat die Bondinglackschicht eine Dicke von weniger als 1 µm. Eine Dicke der Bondinglackschicht im Nanometerbereich ermöglicht außerdem eine Erhöhung der Anzahl der Folienlagen bei gegebener Folienverbundhöhe.

Da der Prägelack für die Auftragung in Schritt (a) vorzugsweise dünnflüssig ist und in einer bevorzugten Ausführungsform die Zusammensetzung des Prägelacks und des Bondinglacks gleich ist, liegt auch der Bondinglack beim Auftragen dünnflüssig vor. In diesem Fall kann es bevorzugt bzw. erforderlich sein, dass das die Trägerfolie mit dem Bondinglack so angeordnet ist, dass die Bondinglackschicht unter Wirkung der Schwerkraft oben auf der Trägerfolie liegt, da anderenfalls ein Verbleib der dünnflüssigen Bondinglackschicht auf der Trägerfolie bis zum Laminierschritt (g) nicht gewährleistet werden kann. In der ersten Ausführungsform werden der Prägelack und der Bondinglack auf dieselbe Flächenseite der Trägerfolie aufgetragen. Diese Flächenseite ist vorzugsweise die Oberseite der Trägerfolie, so dass die genannten Probleme vermieden werden.

Alternativ erfolgt die Anhaftung des Bondinglacks an die Trägerfolie jedoch unabhängig von der Wirkung der Schwerkraft und beruht auf einer nicht-kovalenten Anhaftung. Diese Adhäsion wird durch Einstellen der Oberflächenenergien der Deckfolie und des Bondinglacks, durch die Auswahl eines geeigneten Bondinglackmaterials und/oder insbesondere durch Einsatz einer dünnen Schicht des Bondinglacks erreicht.

Um die Bondinglackschicht viskoser zu machen und sie ggf. kovalent an die Deckfolie zu binden, kann sie teilgehärtet werden. Dies führt aber zu einer geringeren Haftung nach dem Härten in Schritt (h).

In einer Ausgestaltung der ersten Ausführungsform kann die Trägerfolie auf beiden Flächenseiten eine Prägelackschicht aufweisen, wobei jede der beiden Prägelackschichten ihre Prägung auf der dem Träger abgewandten Flächenseite aufweist, so dass eine beidseitig geprägte Struktur erhalten werden kann. Dies kann z. B. durch einen zweimaligen Durchlauf im Rolle-zu-Rolle-Verfahren erfolgen.

### Schritt (c):

Die Prägung von Vertiefungen in die in Schritt (a) erhaltene Prägelackschicht mit einem Prägewerkzeug erfolgt in mindestens einem Teilbereich der Prägelackschicht und umfasst das Bilden einer Vertiefung in der Ebene der Prägelackschicht.

Das Prägen erfolgt mit einem Prägewerkzeug, z. B. einem Stempel, und lässt sich bei Verwendung eines zylindrischen Stempels in einem kontinuierlichen Rolle-zu-Rolle-Verfahren umsetzen.

Es kann die UV-NIL eingesetzt werden, bei der ein nanostrukturierter Stempel mit dem invertierten Profil der gewünschten Struktur als Negativform eingesetzt und in den Prägelack gepresst wird, in dem dann die gewünschte Struktur als Positivform eingeprägt ist. Ein bevorzugtes erfindungsgemäßes Verfahren ist ein Hochdurchsatz-Produktionsverfahren (Rolle-zu-Rolle-UV-NIL) für Mikrofluidikstrukturen, bei dem die Kanäle und Kammern auf großen Polymersubstraten im Rollenformat hergestellt werden.

Die Tiefe der Prägung, also das Ausmaß der Vertiefung in z-Richtung, kann 1 bis weniger als 100 % der Dicke der Prägelackschicht sein. Vorzugsweise ist sie 50 bis weniger als 100 % , stärker bevorzugt 80 bis 100 % der Dicke der Prägelackschicht.

Die Bondinglackschicht kann ebenfalls geprägt werden. Auf diese Weise können sich die Prägungen der Prägelackschicht auf der Trägerfolie und die Prägungen der Bondinglackschicht strukturell und funktionell ergänzen und es können verschiedenste Strukturen mit Mikrofluidikstrukturen und Kavitäten erhalten werden, die nicht erhältlich sind, wenn nur die Prägelackschicht geprägt wird. Die Vertiefungen der Bondinglackschicht können über den Vertiefungen der Prägelackschicht angeordnet sein, so dass ein höherer Hohlraum entsteht. Auf diese Weise können Mikrofluidikstrukturen mit großem Volumen gebildet werden, die beispielsweise als Speicherkammer oder Reaktionskammer eingesetzt werden können.

### Schritt (d):

Das Ausmaß des Teilhärtens der in Schritt (c) geprägten Prägelackschicht wird so eingestellt, dass einerseits eine ausreichende Menge an reaktiven Gruppen für die spätere Härtung und Verbindung mit dem Bondinglack verbleibt und andererseits eine ausreichende Teilhärtung erfolgt, um die erforderliche Stabilität der Strukturen zum Entformen und Falten zu gewährleisten. Dazu werden folgende Parameter optimiert: Polymerisationsgeschwindigkeit des Prägelacks (insbesondere die Art und der Gehalt des Photoinitiators), UV-Intensität, Bahngeschwindigkeit, Transmission der Substratfolie.

### Schritt (e):

Nach dem Prägen und Teilhärten wird das Prägewerkzeug entfernt. Um diesen Schritt zu erleichtern und die Qualität der Prägung zu erhöhen, können dem in Schritt (a) eingesetzten Prägelack oberflächenaktive Antihaftadditive zugesetzt werden.

### Schritt (f):

Die Durchgangsöffnungen werden in z-Richtung durch die in Schritt (e) erhaltene Struktur erzeugt. Beispielsweise erfolgt die Ausbldung von Durchgangsöffnungen an Stellen, an denen geprägte Vertiefungen vorliegen. In Abhängigkeit davon, ob an der jeweiligen Stelle die Trägerfolie einseitig oder beidseitig mit Prägelack überzogen ist und ob die Prägung eine Tiefe von 100 % oder weniger aufweist, muss das Material der Trägerfolie und ggf. Material des Prägelacks entfernt werden. Die Durchgangsöffnungen können mithilfe eines Lasers erzeugt werden.

Die in Schritt (e) erhaltene Struktur wird außerdem vor oder nach der Ausbildung der Durchgangsöffnungen gefaltet. Die Faltung erfolgt vorzugsweise an Faltkanten, die beispielsweise über Laserstrukturierung erzeugt werden. Durch das Ritzen von Faltkanten wird die Selbstausrichtung der Folienlagen ermöglicht.

### Schritt (g)

Die zwei Folienlagen werden aufeinandergelegt. Da es bevorzugt ist, dass die beiden Folienlagen deckungsgleich sind, können die zwei Folienlagen passgenau aufeinandergelegt werden. Bei Bedarf können die zwei Folienlagen bei geringem Druck aneinandergepresst und in diesem Zustand gehalten werden.

Die Prägelackschicht der einen Folienlage wird mit Ausnahme der Vertiefungen der Prägung vollflächig und unmittelbar von der Bondinglackschicht der anderen Folienlage abgedeckt. In den Vertiefungen der Prägung befindet sich vorzugsweise also weder Prägelack noch Bondinglack, so dass Hohlräume entstehen. Die zwei Folienlagen bilden einen sandwichartigen Verbund mit außenliegenden Trägerfolien und innenliegenden Lackschichten, die miteinander verbundenen sind und Mikrofluidikstrukturen aufweisen.

### Schritt (h):

Im erfindungsgemäßen Folienverbund sind die Prägelackschicht und die Bondinglackschicht vollständig gehärtet, also polymerisiert und vernetzt. Diese vollständige Härtung erfolgt in Schritt (h). Die Härtung ist vorzugsweise eine Photopolymerisation und erfolgt durch Einwirken einer Strahlung. Diese Strahlung ist vorzugsweise Licht geeigneter Wellenlänge, beispielsweise UV-Licht. Die Dosis der Strahlung ist so hoch, dass eine vollständige Polymerisierung des Ausgangsmaterials sichergestellt wird. Vorzugsweise wird eine Überdosis eingesetzt, um die Vollständigkeit der Härtung sicherzustellen.

Das Teilhärten in Schritt (d) und das Härten in Schritt (h) können auf gleiche oder unterschiedliche Weise erfolgen, wobei die gleiche Weise bevorzugt ist. Das Teilhärten und das Härten können unabhängig voneinander thermisch oder durch Einsatz von UV-Strahlen oder Elektronenstrahlen erfolgen. Bevorzugt werden das Teilhärten in Schritt (d) und das Härten in Schritt (h) durch UV-Bestrahlung durchgeführt.

### Folienverbund und seine Herstellung gemäß der zweiten Ausführungsform

Die Herstellung von Mikrofluidikstrukturen gemäß der zweiten Ausführungsform ist in WO 2020/187990 A1 beschrieben.

Das Verfahren zum Herstellen eines erfindungsgemäßen Folienverbundes gemäß der zweiten Ausführungsform umfasst die folgenden Schritte:
(a) das Auftragen einer Schicht eines Prägelacks, der zu einem Polymer radikalisch polymerisierbare Monomere enthält, auf eine erste Flächenseite einer Trägerfolie unter Bildung einer Prägelackschicht in einem Prägelackabschnitt,
(b) das Auftragen einer Deckschicht auf die erste Flächenseite der Trägerfolie unter Bildung einer Deckschicht in einem Deckschichtabschnitt,
(c) das Prägen von Vertiefungen in die in Schritt (a) erhaltene Prägelackschicht mit einem Prägewerkzeug,
(d) das Polymerisieren der in der Prägelackschicht enthaltenen Monomere zu einem Polymer unter Bildung einer thermoplastischen Prägelackschicht auf der Trägerfolie ,
(e) das Entfernen des Prägewerkzeugs nach Schritt (d),
(f) das Ausbilden von Durchgangsöffnungen durch die in Schritt (e) erhaltene Struktur von der ersten Flächenseite zu der zweiten Flächenseite der Trägerfolie und das Falten der Struktur zwischen dem Prägelackabschnitt und dem Deckschichtabschnitt unter Bildung von zwei Folienlagen,
(g) das thermische Aneinander-Bonden der in Schritt (f) erhaltenen zwei Folienlagen, so dass die Vertiefungen der Prägelackschicht des Prägelackabschnitts der einen Folienlage von der Deckschicht des Deckschichtabschnitts der anderen Folienlage unter Bildung von Mikrofluidikstrukturen abgeschlossen werden, wobei der Folienverbund erhalten wird.

Vorzugsweise ist die Deckschicht eine thermoplastische Lackschicht, die gemäß den Schritten (c) bis (e) geprägt und polymerisiert wird.

In dem erfindungsgemäßen Folienverbund sind Mikrofluidikstrukturen dadurch ausgebildet, dass Folienlagen mit geprägten Vertiefungen durch die jeweils benachbarte Folienlage bedeckt sind. In der Beschreibung der zweiten Ausführungsform wird zur Veranschaulichung die benachbarte Folienlage bzw. deren unterste Schicht als Deckschicht bezeichnet. Diese Deckschicht befindet sich aber nicht zwingend über der Folienlage mit den geprägten Vertiefungen. Vielmehr kann sie sich in analoger Weise auch unter der Folienlage befinden, wenn die Prägelackschicht auf der Unterseite der Trägerfolie angeordnet ist. Eine Deckschicht kann auch auf beiden Flächenseiten der Folienlage angeordnet sein.

Das Auftragen der Prägelackschicht und der Deckschicht in den Schritten (a) und (b) erfolgt grundsätzlich wie in der ersten Ausführungsform, wobei sich aber die Materialien unterscheiden. Das Prägen in den Schritten (c) bis (e) erfolgt ebenfalls wie in der ersten Ausführungsform beschrieben, wobei sich jedoch in Schritt (d) das Teilhärten des ersten Ausführungsform und das Polymerisieren der zweiten Ausführungsform unterscheiden. Schritt (f) ist in beiden Ausführungsformen gleich.

Das Verfahren der zweiten Ausführungsform unterscheidet sich von dem Verfahren der ersten Ausführungsform im Wesentlichen in der Beschaffenheit der Ausgangsmaterialien der Prägelackschicht und der Bondingschicht bzw. Deckschicht.

Die in der zweiten Ausführungsform eingesetzte Prägelackschicht weist die Merkmale auf, dass es sich um eine Lackschicht handelt, die thermoplastisch, geprägt und radikalisch polymerisiert ist.

In einer Ausgestaltung der zweiten Ausführungsform kann die Trägerfolie auf beiden Flächenseiten eine Prägelackschicht aufweisen, wobei jede der beiden Prägelackschichten ihre Prägung auf der dem Träger abgewandten Flächenseite aufweist, so dass eine beidseitig geprägte Struktur erhalten werden kann. Dies kann z. B. durch einen zweimaligen Durchlauf im Rolle-zu-Rolle-Verfahren erfolgen.

Das Lackschichtausgangsmaterial ist nicht oder nicht vollständig polymerisiert und wird durch radikalische Polymerisation, vorzugsweise Photopolymerisation oder thermische Polymerisation, in den Lack überführt. Es handelt sich dabei also um Monomere oder Oligomere. Es kann auch ein Präpolymer und ein Kettenverlängerer eingesetzt werden.

Das Lackschichtausgangsmaterial wird auf eine Trägerschicht, z. B. ein Polymersubstrat, aufgetragen. Dadurch, dass das Lackschichtausgangsmaterial nicht gehärtet, also nicht polymerisiert ist, weist es eine gewünschte Viskosität auf, so dass es z. B. durch Streichen aufgetragen werden kann. Vorzugsweise ist das Material flüssig und kann somit durch Gießen aufgetragen werden. Im Rolle-zu-Rolle-Verfahren erfolgt das Auftragen des Lackes auf den Träger beispielsweise mittels einer Schlitzdüse oder durch Tiefdruck mit einer Gravurwalze.

Die Viskosität wird so eingestellt, dass das Material leicht aufgetragen werden kann. Andererseits muss es so viskos sein, dass die beim Prägen gebildete Struktur erhalten bleibt.

Es wird eine Lackschicht verwendet, die bei der Polymerisation, z. B. bei Bestrahlung mit UV-Licht, nur oder im Wesentlichen nur lineare Polymerketten bildet. Diese Ketten vernetzen untereinander nicht oder kaum, so dass keine oder nur eine geringe Quervernetzung entsteht. Eine solche Lackschicht zeigt thermoplastisches Verhalten und ist damit thermisch verformbar.

Das Lackschichtausgangsmaterial wird in Schritt (d) vollständig polymerisiert. Die Polymerisation ist vorzugsweise eine Photopolymerisation und erfolgt durch Einwirken einer Strahlung. Diese Strahlung ist vorzugsweise Licht geeigneter Wellenlänge, beispielsweise UV-Licht. Die Dosis der Strahlung ist so hoch, dass eine vollständige Polymerisierung des Ausgangsmaterials sichergestellt wird. Vorzugsweise wird eine Überdosis eingesetzt, um die Vollständigkeit der Polymerisation sicherzustellen.

In dem erfindungsgemäßen Folienverbund gemäß der zweiten Ausführungsform entspricht die Deckschicht der benachbarten Folienlage bzw. ihrer untersten Schicht. Das Deckschichtmaterial kann daher entweder die Trägerfolie der benachbarten Folienlage oder eine darauf aufgebrachte Schicht, beispielsweise eine Prägelackschicht sein. Das Material der Deckschicht ist nicht eingeschränkt und ist vorzugsweise ebenfalls thermoplastisch.

Die Deckschicht ist nach dem Falten der geprägten Flächenseite der Prägelackschicht zugewandt, so dass aufgrund der Vertiefungen in der Prägelackschicht zwischen beiden Schichten Hohlräume befinden, die Mikrofluidikstrukturen ausbilden.

Die Deckschicht kann eine Prägung aufweisen. Auf diese Weise können sich die Prägungen der Prägelackschicht auf der Trägerfolie und die Prägungen der Deckschicht strukturell und funktionell ergänzen und es können verschiedenste Strukturen mit Mikrofluidikstrukturen und Kavitäten erhalten werden, die nicht erhältlich sind, wenn nur die Prägelackschicht geprägt wird. Die Vertiefungen der Deckschicht können über den Vertiefungen der Prägelackschicht angeordnet sein, so dass ein höherer Hohlraum entsteht.

In Schritt (g) erfolgt das thermische Bonden zum Verbinden der Prägelackschicht mit der Deckschicht. Der Ausdruck "thermisches Bonden" bedeutet hier, dass eine Verbindung zwischen zwei Komponenten, z. B. zwei Schichten, durch ein Verfahren in der Art eines thermoplastischen Verschweißens geschaffen wird, welches das Erwärmen mindestens einer der zwei Komponenten umfasst. Beispiele für thermisches Bonden sind das Heißpressen, Heißlaminieren, Thermokompressionsbonden und Ultraschall- sowie Laserschweißen. Das thermische Bonden bedeutet also einerseits, dass die Verbindung zwischen zwei Komponenten durch Erwärmen mindestens einer der zwei Komponenten, Kontaktieren der zwei Komponenten und anschließendes Abkühlen unter Bildung des Verbundmaterials aus den zwei Komponenten hergestellt werden kann, und andererseits, dass diese Verbindung durch Erwärmen zerstörungsfrei auch wieder gelöst werden kann.

Das thermische Bonden zum Verbinden der Prägelackschicht mit der Deckschicht erfolgt bei einer Temperatur (Bonding-Temperatur), bei der die Materialien der beiden Schichten in einem Maße aufgeweicht werden, dass nach dem Abkühlen eine feste Verbindung zwischen den Schichten entsteht. Die Bonding-Temperatur wird in Abhängigkeit von den Materialien der Schichten so ausgewählt, dass einerseits die gewünschte Verbindung der Schichten gebildet wird und andererseits die Prägung in der Prägelackschicht durch das Erweichen nicht beschädigt oder nachteilig verändert wird. Daher sollten die Erweichungstemperaturen der Schichten vorzugsweise ähnlich sein. Am einfachsten kann dies erreicht werden, indem gleiche Materialien für die Prägelackschicht und die Deckschicht mit folglich gleichen Erweichungseigenschaften verwendet werden.

In einer Ausführungsform kann die Prägelackschicht eine etwas höhere Erweichungstemperatur als die Deckschicht aufweisen. Dies kann dazu beitragen, dass beim Erweichen und Bonden die Prägung nicht beschädigt oder verändert wird.

Wenn die Prägelackschicht ausschließlich durch thermisches Bonden an die Deckschicht gebunden ist, werden zwischen den beiden Schichten keine kovalenten Bindungen erzeugt, so dass die beiden Schichten durch Erwärmen auch wieder getrennt werden könnten.

### Anwend ungsbeispiel

Ein Beispiel einer Anwendung des erfindungsgemäßen Folienverbundes ist die in Figur 1 veranschaulichte DNA-Amplifikation mittels isothermischer Amplifikation (LAMP), die den Aufwand der Probenvorbereitung verringert und damit Zeit und Kosten spart. Außerdem wird die Komplexität der Testdurchführung verringert, was den breiten Einsatz als Schnelltest in Arztpraxen und im Privatumfeld erleichtert.

Die für die isothermische Amplifikation benötigte Heizung kann direkt in die nach dem Prägen erzeugten Vertiefungen eingefüllt werden. Beispielsweise kann als Heizmittel eine Metalltinte, z. B. Ag-Nanopartikeltinte, mittels Kapillarwirkung eingefüllt werden. Auf diese Weise können mikrofluidisch gefüllte Elektroden erzeugt werden, die als Folienheizung für die isothermische DNA-Amplifikation genutzt werden können. Je nach Bedarf können die Heizkanäle auf derselben Flächenseite der Folie wie die Mikrofluidikstrukturen oder auf der gegenüberliegenden Flächenseite geprägt werden. Damit wird im Vergleich zur Herstellung einer separaten Heizfolie die Anzahl der Prozessschritte und damit die Kosten deutlich verringert. Gleichzeitig wird die Leistungsfähigkeit gesteigert, da der Wärmeübertrag ohne dazwischenliegende Kleberschichten und zusätzliche Substrate direkter erfolgt und somit weniger Energie verbraucht wird.

Die Herstellung der hierarchischen Strukturen (Kanäle mit mikro- oder nanoskaliger Überstruktur) kann in einem einzigen Prägeschritt stattfinden. Beispielsweise wird die Überstruktur im Rahmen der Masterherstellung gefertigt, entweder durch einen gemeinsamen photolithographischen Schritt oder durch vorangehendes Prägen der Mikro-oder Nanostruktur, und anschließend erfolgt eine Photolithographie über die geprägte Struktur. Beispielsweise erlauben hierarchische Strukturen im Messkanal die Erhöhung der Sensoroberfläche sowie eine Erhöhung der Sensitivität und Selektivität.

### Bezugszeichenliste

- 1: Folienlage
- 2: Trägerfolie
- 3: Prägelackschicht
- 4: Vertiefung
- 5: Mikrofluidikstruktur
- 6: Durchgangsöffnung
- 7, 9: Vertiefung bzw. Mikrofluidikstruktur mit Heizmittel
- 8, 10: Vertiefung bzw. Mikrofluidikstruktur mit Nanostrukturen
- 11: Faltkante
- 12: Hydrophobes Ventil
- 13: Kammer für isothermische PCR
- 14: Mikrostrukturierte Oberfläche
- 15: Mikrokammer mit Heizmittel
- 16: Reaktionskammer
- 17: Verbindungsvertiefung

## Patentansprüche

1. Folienverbund, der aus einer einzelnen Folie unter Bildung von mehreren geschichteten Folienlagen (1) gefaltet ist und Mikrofluidikstrukturen (5) in unterschiedlichen Ebenen umfasst, wobei die Mikrofluidikstrukturen dadurch ausgebildet sind, dass Folienlagen mit geprägten Vertiefungen durch die jeweils in Schichtungsrichtung benachbarte Folienlage bedeckt sind, und wobei Mikrofluidikstrukturen einer Ebene mit Mikrofluidikstrukturen einer anderen Ebene über Durchgangsöffnungen in mindestens einer Folienlage verbunden sind.

2. Folienverbund nach Anspruch 1, der fächerförmig gefaltet ist.

3. Folienverbund nach Anspruch 1 oder 2, wobei mindestens eine der Folienlagen eine Trägerfolie und auf einer Flächenseite der Trägerfolie eine Lackschicht mit geprägten Vertiefungen aufweist; und/oder wobei mindestens eine der Folienlagen eine Trägerfolie und auf beiden Flächenseiten der Trägerfolie eine Lackschicht mit geprägten Vertiefungen auf mindestens einer der beiden Flächenseite der Trägerfolie aufweist.

4. Folienverbund nach einem der vorstehenden Ansprüche, wobei die Folie an Faltkanten gefaltet ist, die durch Laserschneiden der Folien erzeugt wurden, und/oder die Durchgangsöffnungen durch Laserschneiden erzeugt wurden.

5. Folienverbund nach einem der vorstehenden Ansprüche, der aus deckungsgleichen Folienlagen, die vollflächig unmittelbar übereinanderliegen, und Faltkanten besteht.

6. Folienverbund nach einem der vorstehenden Ansprüche, wobei mindestens eine der Folienlagen eine Trägerfolie und mindestens eine Lackschicht mit Vertiefungen aufweist, die sich bis zur Trägerfolie erstrecken, so dass die gebildete Mikrofluidikstruktur teilweise von der Trägerfolie bedeckt wird.

7. Folienverbund nach einem der vorstehenden Ansprüche, der in den Mikrofluidikstrukturen mindestens eine Modifikation aufweist, die unter einer Funktionalisierung, Nanostrukturierung, Hydrophobisierung und Heizmittelfüllung ausgewählt ist.

8. Folienverbund nach einem der vorstehenden Ansprüche, wobei mindestens eine der Folienlagen eine Trägerfolie und auf mindestens einer Flächenseite der Trägerfolie eine Lackschicht mit geprägten Vertiefungen aufweist, wobei entweder die Vertiefungen in einer Lackschicht der Folienlagen vorliegen und die Lackschicht durch UV-Härten mit der benachbarten Folienlage verbunden ist oder die Vertiefungen in einer thermoplastischen Lackschicht der Folienlagen vorliegen und die Lackschicht durch ausschließlich thermisches Bonden mit der benachbarten Folienlage verbunden ist.

9. Folienverbund nach Anspruch 1, der fächerförmig gefaltet ist und aus deckungsgleichen Folienlagen, die vollflächig unmittelbar übereinanderliegen, und Faltkanten besteht, wobei die Faltkanten und die Durchgangsöffnungen unter Einsatz eines Lasers erzeugt wurden und wobei die Folienlagen eine Trägerfolie und eine Lackschicht mit Vertiefungen aufweisen und die Lackschicht durch UV-Härten mit der benachbarten Folienlage verbunden ist.

10. Verfahren zum Herstellen eines Folienverbundes nach einem der Ansprüche 1 bis 9, welches die folgenden Schritte umfasst:
(a) das Auftragen einer Schicht eines härtbaren Prägelacks auf eine erste Flächenseite einer Trägerfolie unter Bildung einer Prägelackschicht in einem Prägelackabschnitt,
(b) das Auftragen einer Schicht eines härtbaren Bondinglacks auf die erste Flächenseite der Trägerfolie unter Bildung einer Bondinglackschicht in einem Bondinglackabschnitt,
(c) das Prägen von Vertiefungen in die in Schritt (a) erhaltene Prägelackschicht mit einem Prägewerkzeug,
(d) das Teilhärten der in Schritt (c) geprägten Prägelackschicht,
(e) das Entfernen des Prägewerkzeugs nach Schritt (d),
(f) das Ausbilden von Durchgangsöffnungen durch die in Schritt (e) erhaltene Struktur von der ersten Flächenseite zu der zweiten Flächenseite der Trägerfolie und das Falten der Struktur zwischen dem Prägelackabschnitt und dem Bondinglackabschnitt unter Bildung von zwei Folienlagen,
(g) das Aufeinanderlegen der in Schritt (f) erhaltenen zwei Folienlagen unter Bildung eines Verbundes, so dass die Vertiefungen der Prägelackschicht des Prägelackabschnitts der einen Folienlage von der Bondinglackschicht des Bondinglackabschnitts der anderen Folienlage unter Bildung von Mikrofluidikstrukturen abgeschlossen werden,
(h) das Härten der teilgehärteten Prägelackschicht und der Bondinglackschicht des in Schritt (g) erhaltenen Verbundes unter Ausbildung kovalenter Bindungen zwischen diesen Schichten, wobei der Folienverbund erhalten wird.

11. Verfahren nach Anspruch 10, wobei der härtbare Bondinglack ein härtbarer Prägelack ist, der gemäß den Schritten (c) bis (e) geprägt und teilgehärtet wird.

12. Verfahren zum Herstellen eines Folienverbundes nach einem der Ansprüche 1 bis 9, welches die folgenden Schritte umfasst:
(a) das Auftragen einer Schicht eines Prägelacks, der zu einem Polymer radikalisch polymerisierbare Monomere enthält, auf eine erste Flächenseite einer Trägerfolie unter Bildung einer Prägelackschicht in einem Prägelackabschnitt,
(b) das Auftragen einer Deckschicht auf die erste Flächenseite der Trägerfolie unter Bildung einer Deckschicht in einem Deckschichtabschnitt,
(c) das Prägen von Vertiefungen in die in Schritt (a) erhaltene Prägelackschicht mit einem Prägewerkzeug,
(d) das Polymerisieren der in der Prägelackschicht enthaltenen Monomere zu einem Polymer unter Bildung einer thermoplastischen Prägelackschicht auf der Trägerfolie ,
(e) das Entfernen des Prägewerkzeugs nach Schritt (d),
(f) das Ausbilden von Durchgangsöffnungen durch die in Schritt (e) erhaltene Struktur von der ersten Flächenseite zu der zweiten Flächenseite der Trägerfolie und das Falten der Struktur zwischen dem Prägelackabschnitt und dem Deckschichtabschnitt unter Bildung von zwei Folienlagen,
(g) das thermische Aneinander-Bonden der in Schritt (f) erhaltenen zwei Folienlagen, so dass die Vertiefungen der Prägelackschicht des Prägelackabschnitts der einen Folienlage von der Deckschicht des Deckschichtabschnitts der anderen Folienlage unter Bildung von Mikrofluidikstrukturen abgeschlossen werden, wobei der Folienverbund erhalten wird.

13. Verfahren nach Anspruch 12, wobei die Deckschicht eine thermoplastische Prägelackschicht ist, die gemäß den Schritten (c) bis (e) geprägt und polymerisiert wird.

14. Verfahren nach einem der Anspüche 10 bis 13, wobei das Prägen in einem Rolle-zu-Rolle-Verfahren durchgeführt wird.

15. Verwendung eines Folienverbundes nach einem der Ansprüche 1 bis 9 für die Amplifikation von DNA durch Polymerasekettenreaktion oder isothermische Amplifikation.
